# EUROPEAN PATENT SPECIFICATION

(11) **EP 3 558 269 B1**
(45) Date of publication and mention of the grant of the patent: **04.09.2024**
(21) Application number: 16829317.3
(22) Date of filing: 21.12.2016
(51) Int. Cl.: A61K 9/107, A61K 47/44, A61K 31/167

(54) **A METHOD OF MANUFACTURING A WATER-IN-OIL EMULSION OF NANOPARTICLES OF PARACETAMOL**
VERFAHREN ZUR HERSTELLUNG EINER WASSER-IN-ÖL-EMULSION VON PARACETAMOL-NANOPARTIKELN
MÉTHODE DE FABRICATION D'UNE ÉMULSION EAU DANS HUILE DE NANOPARTICULES DE PARACÉTAMOL

(43) Date of publication of application: 30.10.2019
(73) Proprietor: Dukebox SP. Z O.O., 01-059 Warszawa (PL)
(72) Inventor: BANACH, Marcin, 32-100 Proszowice (PL); PULIT-PROCIAK, Jolanta, 31-866 Krakow (PL)
(74) Representative: Krekora, Magdalena
(86) International application number: PCT/IB2016/057901
(87) International publication number: WO 2018/115932

(56) References cited:
- WO-A1-2012/075534
- WO-A1-2015/198350
- WO-A2-2014/106048
- AU-A1- 2006 309 295
- US-A1- 2014 322 330

## Description

### TECHNICAL FIELD

The object of the present invention is a method of manufacturing a water-in-oil emulsion of nanoparticles of paracetamol.

### BACKGROUND ART

Paracetamol, also known as acetaminophen or APAP, is a medication used to treat pain and fever. Paracetamol is used to treat many conditions such as headache, muscle aches, arthritis, backache, toothaches, colds, and fevers. It relieves pain in mild arthritis but has no effect on the underlying inflammation and swelling of the joint.

Patent application US20060147538A1 discloses a drug delivery composition comprising an active ingredient and a biologically inert material wherein the biologically inert material is a nanocomposite material. Preferably the biologically inert material is a polymer-clay nanocomposite comprising up to about 40% by weight of nano-sized (1-1000 nm) clay particles dispersed in a polymeric material. The active ingredient may be dispersed in the nanocomposite material or absorbed thereto.

Patent application CN102218019A1 discloses a preparation method for a nano-granular solid dispersion of a hydrophobic drug by high-voltage electrostatic spraying, which comprises the following steps of: mixing a hydrophobic drug, a drug-carrying polymer and an organic solvent at a mass ratio of (1-10): (10-20): (80-94) to prepare a consolute electric spraying solution; and then carrying out high-voltage electrostatic spraying while controlling the flow rate of the consolute electric spraying solution at 0.5-2.5 mL/h, the distance between a receiving board and a wire spraying port at 15-30cm and the voltage at 5-30kV to obtain the nano-granular solid dispersion of the hydrophobic drug. The preparation method is simple to operate, is low in cost, is environmentally-friendly and is suitable for industrial production; and the prepared solid dispersion of the hydrophobic drug not only can highly disperse a composite of a polymer and the drug into an amorphous state and has a nano-structural characteristic.

Patent application WO2007053197A1 discloses a stable natioparticulate acetaminophen composition comprising:
(a) particles of acetaminophen or a salt or derivative thereof having an effective average particle size of less than about 2000 nm; and
(b) at least one surface stabilizer, castor oil derivatives.

The composition may contain castor oil or fatty acid esters of sorbitan as emulsifier.

Patent application EP2938324A1 discloses a compositions containing acetaminophen, wherein the active ingredient is stabilized and greater than 90% of the particles of the active ingredient have a particle size that is less than or equal to or any number in between 100, 90, 80, 70, 60, 50, 40, 30, 20, 19, 18, 17, 16, 15, 14, 13, 12, 11, 10, 9, 8, 7, 6, 5, or 4 nanometers, or smaller, as determined by Dynamic Light Scattering (DLS), using a volume -weighted particle size distribution calculation method.

Patent application US20140322330A1 relates to methods of forming miniemulsions and use of the miniemulsions as a delivery system for bioactive agents. In particular, it relates to methods for forming a miniemulsion comprising providing a first phase comprising a hydrophilic surfactant, lipophillic surfactant and water and a second phase comprising a lipid, wherein said miniemulsion comprises emulsified particles having a mean diameter of 1 µm or less.

Patent application WO2015198350A1 discloses a method of obtaining a pharmaceutical oil-in-water nano-emulsion, with a pharmaceutically active substance. The selected pharmaceutically active substance e.g. paracetamol is encased in monounsaturated fatty acid droplets with the droplets having an average particle size in the range of 60 to 200 nm. The nano-emulsion is also provided with a non-ionic surfactant system, which is a mixture of polyethers, macrogolglycerides and polysaccharides, along with pharmaceutically acceptable adjuvants. The present invention also provides a process for the preparation of pharmaceutical oil-in-water nano-emulsion.

### DISCLOSURE OF THE INVENTION

The method in accordance with the present invention solves the problem of preparation of stable water-in-oil nanoemulsions of paracetamol and thus improving their bioavailability and efficacy.

A method of manufacturing a water-in-oil emulsion of nanoparticles of paracetamol of the present invention consists in that, an oil phase is prepared by dissolution of paracetamol in castor oil, and a water phase is prepared by dissolution of at least one emulsifier/stabilizer and possibly of potassium sorbate in water, where the emulsifier/stabilizer is chosen from a group consisting of lecithin, gelatin, and starch, then the oil phase is added dropwise to the water phase, then the two-phase system is homogenised from 10 to 45 minutes through the use of ultrasonic frequencies, where the strength of ultrasound is from 500 to 600 W and ultrasonication is carried out in flow system or in batch system, until an emulsion is obtained.

Preferably the concentration of paracetamol in oil is from 9 to 39% w/w.

Preferably the concentration of lecithin in water phase is from 5 to 10%w/w.

Preferably the concentration of gelatine in water phase is from 0.5 to 2%w/w.

Preferably, the concentration of starch in water phase is from 1 to 2%w/w.

Preferably the concentration of potassium sorbate in water phase is from 0.4 to 0.8%w/w.

Preferably the emulsifier/stabilizer is dissolved in water in temperature not higher than do 90°C.

Preferably the mass ratio of oil phase to water phase is from 0.8:1.0 to 1.0:1.0.

The present invention refers also to a water-in-oil emulsion of paracetamol nanoparticles obtained by method according to the invention.

### BEST MODE OF CARRYING OUT THE INVENTION

The following examples illustrate the invention without setting or delineating its limits.

### Example 1

A solution of paracetamol in castor oil was prepared by mixing 30 g of paracetamol with 143.6 g of castor oil. 10 g of lecithin, and 2 g of starch were dissolved in 150 g of water in temperature 80°C. Then, during continuous homogenization, the solution of paracetamol was added portion-wise to the solution of lecithin, and starch. After the whole oil-phase is added, homogenization is continued for 15 minutes. A homogeneous water-in-oil emulsion is obtained comprising paracetamol in the amount of 100 mg/cm³.

The Malvern Zetasizer apparatus using method of dynamic light scattering (DLS) was utilized to measure the average size of paracetamol particles. The average size of nanoparticles was 69 nm.

### Example 2

A solution of paracetamol in castor oil was prepared by mixing 15 g of paracetamol with 143.6 g of castor oil. 10 g of lecithin, 2 g of gelatine, 2 g of starch, and 0.8 g of potassium sorbate were dissolved in 150 g of water in temperature 80°C. Then, during continuous homogenization, the solution of paracetamol was added portion-wise to the solution of emulsifiers/stabilizers. After the whole oil-phase is added, homogenization is continued for 20 minutes. A homogeneous water-in-oil emulsion is obtained comprising paracetamol in the amount of 50 mg/cm³.

The Malvern Zetasizer apparatus using method of dynamic light scattering (DLS) was utilized to measure the average size of paracetamol particles. The average size of nanoparticles was 119 nm. The size of particles was then measured after 48 hours. It was equal to 112 nm.

### Example 3

A solution of paracetamol in castor oil was prepared by mixing 30 g of paracetamol with 143.6 g of castor oil. 10 g of lecithin, 1 g of gelatine, and 0.8 g of potassium sorbate were dissolved in 150 g of water in temperature 80°C. Then, during continuous homogenization, the solution of paracetamol was added portion-wise to the solution of emulsifiers/stabilizers. After the whole oil-phase is added, homogenization is continued for 15 minutes. A homogeneous water-in-oil emulsion is obtained comprising paracetamol in the amount of 100 mg/cm³.

The Malvern Zetasizer apparatus using method of dynamic light scattering (DLS) was utilized to measure the average size of paracetamol particles. The average size of nanoparticles was 55 nm. The size of particles was then measured after 48 hours. It was equal to 36 nm.

Potential zeta was equal to -23 mV. The absolute value of the electrokinetic (zeta) potential confirms high stability of the solution.

## Claims

1. A method of manufacturing a water-in-oil emulsion of nanoparticles of paracetamol, **characterised in that**, an oil phase is prepared by dissolution of paracetamol in castor oil, and a water phase is prepared by dissolution of at least one emulsifier/stabilizer and possibly of potassium sorbate in water, where the emulsifier/stabilizer is chosen from a group consisting of lecithin, gelatin, and starch, then the oil phase is added dropwise to the water phase, then the two-phase system is homogenised from 10 to 45 minutes through the use of ultrasonic frequencies, where the strength of ultrasound is from 500 to 600 W and ultrasonication is carried out in flow system or in batch system, until an emulsion is obtained.

2. A method according to claim 1, **characterized in that**, the concentration of paracetamol in oil is from 9 to 39%w/w.

3. A method according to claim 1 or 2, **characterized in that**, the concentration of lecithin in water phase is from 5 to 10%w/w.

4. A method according to any of the preceding claims, **characterized in that**, the concentration of gelatine in water phase is from 0.5 to 2%w/w.

5. A method according to any of the preceding claims, **characterized in that**, the concentration of starch in water phase is from 1 to 2% w/w.

6. A method according to any of the preceding claims, **characterized in that**, the concentration of potassium sorbate in water phase is from 0.4 to 0.8% w/w.

7. A method according to any of the preceding claims, **characterized in that**, the emulsifier/stabilizer is dissolved in water in temperature not higher than do 90°C.

8. A method according to any of the preceding claims, **characterized in that**, the mass ratio of oil phase to water phase is from 0.8:1.0 to 1.0:1.0.

9. A water-in-oil emulsion of paracetamol nanoparticles obtained by method according to any of the preceding claims.

## Patentansprüche

1. Verfahren zur Herstellung einer Wasser-in-Öl-Emulsion von Paracetamol-Nanopartikeln, **dadurch gekennzeichnet, dass** eine Ölphase durch Auflösen von Paracetamol in Rizinusöl und eine Wasserphase durch Auflösen von mindestens einem Emulgator/Stabilisator und gegebenenfalls von Kaliumsorbat in Wasser hergestellt wird, wobei der Emulgator/Stabilisator aus einer Gruppe ausgewählt wird, die aus Lecithin, Gelatine und Stärke ausgewählt wird, dann die Ölphase tropfenweise zu der Wasserphase gegeben wird, dann das Zweiphasensystem 10 bis 45 Minuten lang unter Verwendung von Ultraschallfrequenzen homogenisiert wird, wobei die Ultraschallstärke 500 bis 600 W beträgt und die Ultraschallbehandlung in einem Durchflusssystem oder in einem Chargensystem durchgeführt wird, bis eine Emulsion erhalten wird.

2. Verfahren nach Anspruch 1, **dadurch gekennzeichnet, dass** die Konzentration von Paracetamol in Öl 9 bis 39 Gew.-% beträgt.

3. Verfahren nach Anspruch 1 oder 2, **dadurch gekennzeichnet, dass** die Konzentration von Lecithin in der Wasserphase 5 bis 10 Gew.-% beträgt.

4. Verfahren nach einem der vorstehenden Ansprüche, **dadurch gekennzeichnet, dass** die Konzentration von Gelatine in der Wasserphase 0,5 bis 2 Gew.-% beträgt.

5. Verfahren nach einem der vorstehenden Ansprüche, **dadurch gekennzeichnet, dass** die Konzentration von Stärke in der Wasserphase 1 bis 2 Gew.-% beträgt.

6. Verfahren nach einem der vorstehenden Ansprüche, **dadurch gekennzeichnet, dass** die Konzentration von Kaliumsorbat in der Wasserphase 0,4 bis 0,8 Gew.-% beträgt.

7. Verfahren nach einem der vorhergehenden Ansprüche, **dadurch gekennzeichnet, dass** der Emulgator/Stabilisator in Wasser bei einer Temperatur von nicht mehr als 90 °C gelöst wird.

8. Verfahren nach einem der vorhergehenden Ansprüche, **dadurch gekennzeichnet, dass** das Massenverhältnis von Ölphase zu Wasserphase 0,8:1,0 bis 1,0:1,0 beträgt.

9. Wasser-in-Öl-Emulsion von Paracetamol-Nanopartikeln, die durch ein Verfahren gemäß einem der vorstehenden Ansprüche erhalten wurde.

## Revendications

1. Une méthode de fabrication d'une émulsion eau dans huile de nanoparticules de paracétamol, **caractérisée en ce qu'**une phase huileuse est préparée par dissolution du paracétamol dans de l'huile de ricin, et une phase aqueuse est préparée par dissolution d'au moins un émulsifiant/stabilisant et éventuellement du sorbate de potassium dans l'eau, où l'émulsifiant/stabilisant est choisi dans un groupe constitué de lécithine, de gélatine et d'amidon, puis la phase huileuse est ajoutée goutte à goutte à la phase aqueuse, puis le système biphasique est homogénéisé de 10 à 45 minutes par l'utilisation de fréquences ultrasonores, où la force des ultrasons est de 500 à 600 W et l'ultrasonication est réalisée en système à écoulement ou en système par lots, jusqu'à obtention d'une émulsion.

2. Une méthode selon la revendication 1, **caractérisée en ce que** la concentration de paracétamol dans l'huile est de 9 à 39% en poids.

3. Une méthode selon la revendication 1 ou 2, **caractérisée en ce que** la concentration de lécithine dans la phase aqueuse est de 5 à 10% en poids.

4. Une méthode selon l'une quelconque des revendications précédentes, **caractérisée en ce que** la concentration de gélatine dans la phase aqueuse est de 0,5 à 2 % en poids.

5. Une méthode selon l'une quelconque des revendications précédentes, **caractérisée en ce que** la concentration d'amidon dans la phase aqueuse est de 1 à 2 % en poids.

6. Une méthode selon l'une quelconque des revendications précédentes, **caractérisée en ce que** la concentration de sorbate de potassium dans la phase aqueuse est de 0,4 à 0,8 % en poids.

7. Une méthode selon l'une quelconque des revendications précédentes, **caractérisée en ce que** l'émulsifiant/stabilisant est dissous dans de l'eau à une température pas plus élevée que 90°C.

8. Une méthode selon l'une quelconque des revendications précédentes, **caractérisée en ce que** le rapport massique de la phase huileuse à la phase aqueuse est de 0,8:1,0 à 1,0:1,0.

9. Une émulsion eau dans huile de nanoparticules de paracétamol obtenue par une méthode selon l'une quelconque des revendications précédentes.
